# FASCICULE DE BREVET EUROPEEN

(11) **EP 1 408 812 B1**
(45) Date de publication et mention de la délivrance du brevet: **07.06.2006**
(21) Numéro de dépôt: 02790205.5
(22) Date de dépôt: 19.07.2002
(51) Int. Cl.: A61B 1/00

(54) **DISPOSITIF DE LIAISON INTERVERTEBRAL**
WIRBELVERBINDUNGSVORRICHTUNG
INTERVERTEBRAL LINKING DEVICE

(30) Priorité: 20.07.2001 FR 0109773
(43) Date de publication de la demande: 21.04.2004
(73) Titulaire: SDGI Holdings, Inc., Wilmington, DE 19801 (US)
(72) Inventeur: GRAF, Henry, 69006 Lyon (FR)
(74) Mandataire: Neyret, Daniel Jean Marie
(86) Numéro de dépôt international: PCT/FR2002/002593
(87) Numéro de publication internationale: WO 2003/009737

(56) Documents cités:
- EP-A- 0 897 697
- WO-A-02/00124
- WO-A-95/35067
- WO-A-99/05968
- US-A- 5 876 402

## Description

La présente invention concerne un dispositif de liaison intervertébral.

On connaît un tel dispositif qui comprend au moins deux vis pédiculaires, dont chacune possède une première extrémité solidarisée à un corps vertébral correspondant, une portion intermédiaire renflée, ainsi qu'une seconde extrémité filetée. Des organes auxiliaires, pourvus d'une arche de fixation d'une tige s'étendant entre les vertèbres, sont disposée sur chacune des portions renflées précitées. Un boulon, coopérant avec l'extrémité filetée de chaque vis, permet l'immobilisation de chaque organe auxiliaire, une fois ce dernier mis en place de façon appropriée. Ce dispositif est décrit dans US-A-6 019 759.

Ce dispositif connu présente cependant certains inconvénients, en ce sens qu'il implique un procédé de montage relativement délicat. Par ailleurs, une fois implanté, il n'offre aucun degré de liberté entre les différents éléments qui le constituent. Ainsi, lorsque des efforts s'exercent au niveau des corps vertébraux, cette absence de degré de liberté induit une transmission de ces efforts sur l'ensemble du dispositif, de sorte que ce dernier a tendance à se désolidariser des vertèbres qu'il relie et induit par ailleurs des dysfonctionnements au niveau de l'ensemble de la chaîne vertébrale.

Afin de pallier ces différents inconvénients, la présente invention se propose de réaliser un dispositif dont la structure est simple, dont le montage est aisé et qui est implanté de façon fiable dans les vertèbres qu'il relie.

Le document EP-A-0 897 697 présente les caractéristiques figurant dans le préambule de la revendication 1.

A cet effet, elle a pour objet un dispositif de liaison intervertébral, destiné à relier au moins deux vertèbres entre elles, comprenant :
- au moins un élément fixe, destiné à être rendu solidaire d'une vertèbre ou du sacrum,
- au moins un élément mobile de liaison, apte à se déplacer par rapport au ou à chaque élément fixe,
- ainsi qu'au moins un élément intermédiaire, permettant l'articulation du ou de chaque élément mobile par rapport au ou à chaque élément fixe,
- le ou chaque élément intermédiaire étant reçu, en service, dans un volume intérieur de l'élément mobile, ou bien de l'élément fixe, cet élément intermédiaire étant déformable, de manière à pouvoir être introduit par impaction dans ce volume intérieur,
- l'élément fixe, ou bien l'élément mobile, étant reçu au moins partiellement, en service, dans un volume intérieur de l'élément intermédiaire, caractérisé en ce que l'élément fixe ou bien l'élément mobile présente, avec l'élément intermédiaire, une position mutuelle d'utilisation, dans laquelle cet élément fixe ou bien cet élément mobile possède trois denrée de liberté en rotation, mais se trouve lié en translation, par rapport à l'élément intermédiaire, et une position mutuelle d'introduction, dans laquelle cet élément fixe, ou bien cet élément mobile, possède trois degrés de liberté en rotation et en translation par rapport à cet élément intermédiaire, ladite introduction étant permise par un méplat équatorial ménagé sur une tête sphérique de l'élément fixe, ou de l'élément mobile, ledit méplat équatorial n'étant pas perpendiculaire à l'axe principal de l'élément fixe, ou de l'élément mobile.

Selon d'autres caractéristiques de l'invention :
- l'élément intermédiaire affecte la forme d'une cupule ;
- le volume intérieur de la cupule intermédiaire est bordé par une surface sphérique tronquée
- la cupule intermédiaire possède une surface extérieure sphérique tronquée, concentrique à la surface intérieure ;
- les surfaces intérieure et extérieure définissent une paroi de la cupule intermédiaire ;
- l'épaisseur de la paroi est comprise entre 0,5 et 3 mm, de préférence entre 1 et 1,5 mm ;
- l'élément intermédiaire est réalisé en polyéthylène

L'invention va être décrite ci-dessous, en référence aux dessins annexés, donnés uniquement à titre d'exemples non limitatifs, dans lesquels :
- La figure 1 est une vue en coupe longitudinale, illustrant les différents éléments constitutifs d'un dispositif de liaison intervertébral conforme à l'invention ;
- Les figures 2A, 2B et 2C sont des vues analogues à la figure 1, illustrant deux étapes du montage d'un élément intermédiaire du dispositif de la figure 1, dans le volume intérieur d'un élément mobile de ce dispositif ; et
- Les figures 3A et 3B sont des vues analogues à la figure 1, illustrant l'introduction d'un élément fixe du dispositif de la figure 1, dans le volume intérieur de l'élément intermédiaire.

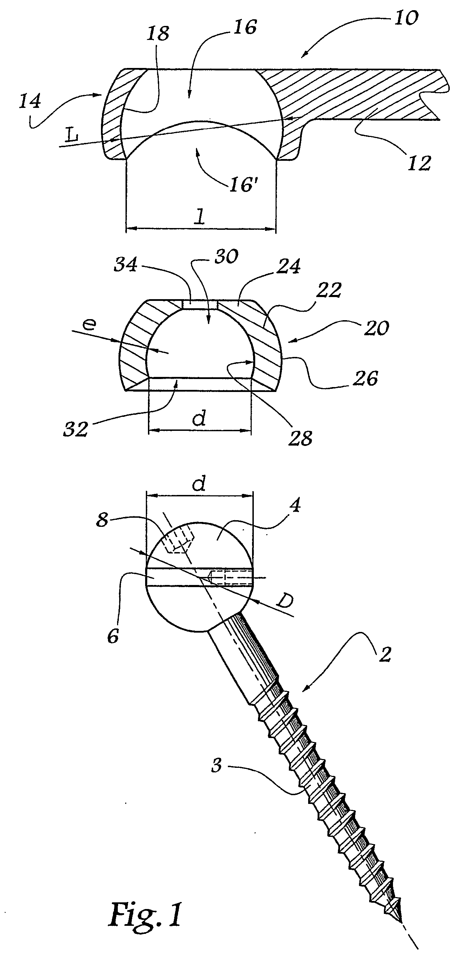

Le dispositif de liaison illustré à la figure 1 comprend une vis pédiculaire 2, destinée à être solidarisée dans un corps vertébral non représenté.

Cette vis pédiculaire, qui constitue un élément fixe du dispositif de liaison, est pourvue d'une tige 3 terminée par une tête sphérique 4, qui comporte un méplat équatorial 6. Ce dernier s'étend de façon inclinée, en ce sens qu'il n'est pas perpendiculaire à l'axe principal A de la vis 2.

La tête 4 est en outre creusée, à l'opposé de la tige 3, d'un trou borgne 8. Ce dernier est destiné à la réception d'un organe de manoeuvre non représenté, qui est par exemple l'extrémité d'un tournevis ou d'une clé hexagonale.

Le dispositif de liaison de la figure 1 comprend également un élément mobile, illustré partiellement, qui est désigné dans son ensemble par la référence 10. Cet élément possède un corps 12, qui s'étend entre les deux vertèbres devant être reliées par le dispositif de l'invention. Ce corps est terminé par deux extrémités creuses, dont une seule 14 est représentée.

Chaque extrémité définit un logement 16, constituant un volume intérieur de l'élément 10, qui est bordé par des parois 18 formant un tronçon de sphère. La dimension transversale l du débouché 16' du logement 16 est inférieure au diamètre L de ce logement.

Le dispositif de la figure 1 comprend enfin un élément intermédiaire 20, qui constitue une cupule. Cette dernière, qui présente une forme hémisphérique tronquée, possède une paroi mince 22, qui s'étend à partir d'un fond 24 de cette cupule.

La surface extérieure 26 de la paroi 22 délimite une portion de sphère, dont le diamètre est identique à celui L du logement 16. Par ailleurs, la surface intérieure 28 de la paroi 22, qui forme un volume intérieur 30 de la cupule 20, possède un diamètre D identique à celui de la tête 4.

En outre, la dimension transversale d du débouché 32 du volume intérieur 30 est égale à celle du méplat 6 de la tête 4. Ce débouché est plus « étroit » que le volume intérieur, dans la mesure où la surface intérieure 28 sphérique s'étend selon un angle de plus de 180°.

Enfin, le fond 24 de la cupule 20 est creusé d'une ouverture 34, permettant le passage d'un outil de manoeuvre en direction du trou borgne 8 de la vis pédiculaire 2.

Il est à noter que la cupule 20 est réalisée en un matériau déformable, tel que du polyéthylène. Cette caractéristique permet, en association avec la minceur de la paroi 22, d'assurer une introduction par impaction de la cupule 20 dans le logement 16 de l'élément mobile 10. L'épaisseur e de cette paroi 22 est par exemple comprise entre 0,5 et 3 mm, de préférence entre 1 et 1,5 mm.

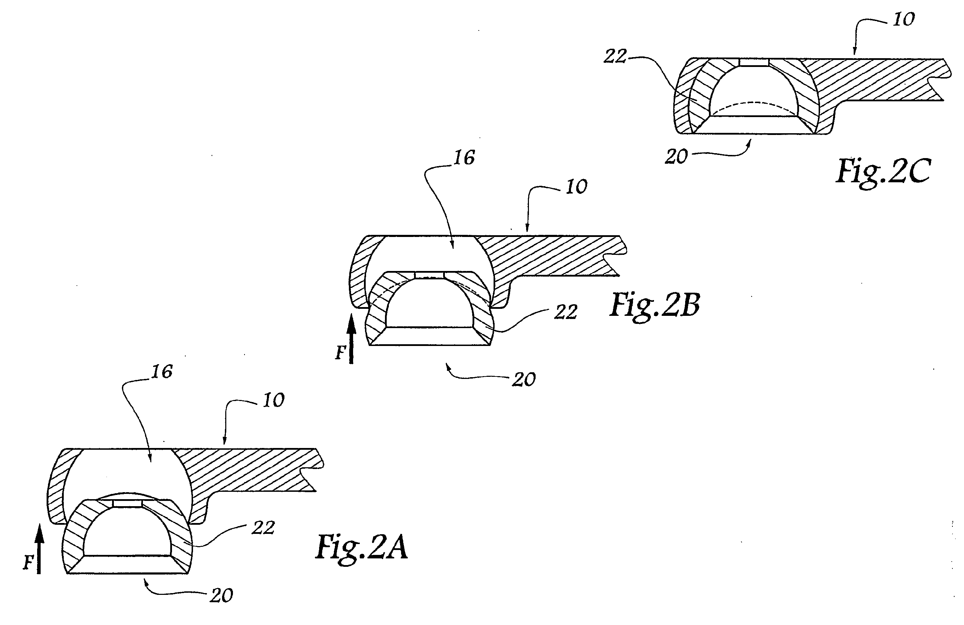

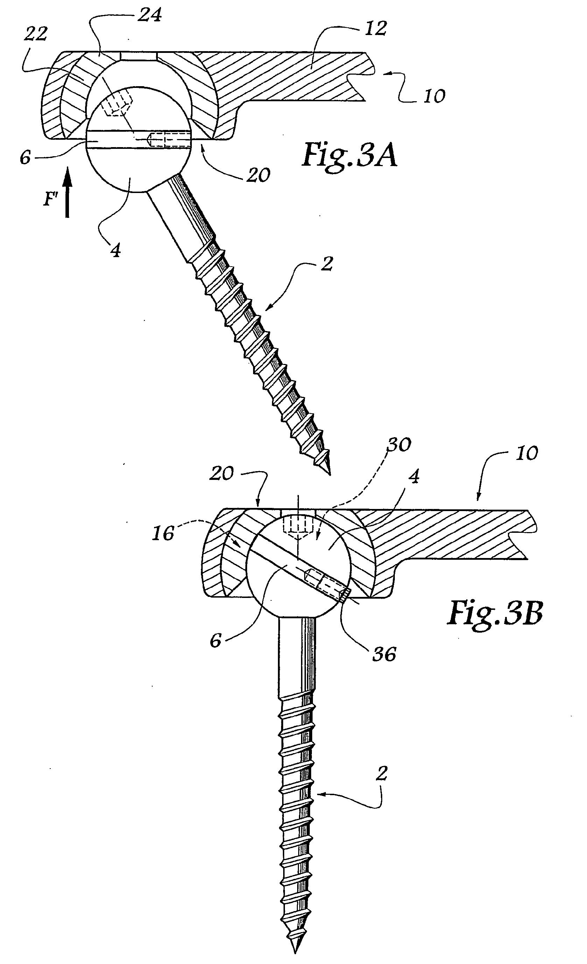

Le montage du dispositif de liaison illustré à la figure 1, va maintenant être décrit en référence aux figures 2A, 2B, 2C, 3A et 3B.

Il s'agit, dans un premier temps, d'introduire la cupule 20 dans le volume intérieur 16 de l'élément mobile 10.

A cet effet, comme le montre la figure 2A, on dispose la cupule 20 de manière qu'elle se trouve en regard du logement 16. Puis, on la rapproche axialement de l'élément mobile 10, selon la flèche F.

Etant donné que cette cupule 20 est déformable élastiquement, ses dimensions transversales, en particulier le diamètre intérieur D de sa paroi 22, sont aptes à subir une diminution momentanée. Ceci permet donc d'introduire la cupule 20, par impaction selon la flèche F, dans le logement 16 de l'élément mobile 10 (figure 2B).

Une fois cette opération réalisée, comme le montre là figure 2C, la surface extérieure 26 de la paroi mince 22 s'étend au contact de la surface intérieure 18 du logement 16, de même diamètre. Ainsi, la cupule 20 possède trois degrés de liberté en rotation par rapport à l'élément mobile.

En revanche, elle ne possède aucun degré de liberté en translation par rapport à cet élément 10, dans cette position d'utilisation. En effet, le pourtour du débouché 16', dont la dimension transversale est inférieure au diamètre du logement 16, empêche la cupule 20 de ressortir de ce logement.

Puis, il s'agit d'introduire la tête sphérique 4 de la vis 2 dans le logement 30 de la cupule 20.

A cet effet, on incline tout d'abord cette vis 2, de sorte que le méplat 6 s'étende horizontalement sur la figure 3A, à savoir perpendiculairement à l'axe principal de la cupule 20. On rapproche alors la cupule 20 de la vis 2, selon une translation parallèle à l'axe principal de cette cupule 20 (flèche F').

Etant donné que la dimension transversale du méplat est égale à celle d du débouché 32 du logement 30, ceci permet une libre introduction de la tête 4 dans ce logement.

Ensuite, on fait pivoter la tête 4 à l'intérieur du logement, de manière que le méplat 6 ne se trouve plus en regard du débouché 32 précité. Dans cette position d'utilisation (figure 3B), la tête 4 se trouve libre de pivoter par rapport au logement 30, mais ne possède aucun degré de liberté en translation par rapport à la cupule 20.

En effet, le diamètre D de la tête 4 est supérieur à la dimension transversale du débouché 32. Par ailleurs, le pourtour de ce dernier est rendu sensiblement rigide, du fait de la présence des parois rigides de l'élément mobile 10. Ainsi, le pourtour de ce débouché 32 ne peut quasiment pas se déformer radialement, ce qui empêche la tête 4 de ressortir du volume intérieur 30.

Une fois le dispositif placé dans la configuration de la figure 3B, il s'agit de fixer la vis pédiculaire 2 dans un corps vertébral correspondant, au moyen d'un organe de manoeuvre coopérant avec le trou borgne 8.

A titre de variante de montage, il est possible de fixer tout d'abord chaque vis pédiculaire dans un corps vertébral correspondant. Puis, on introduit chaque cupule 20 dans un volume intérieur 16 correspondant de l'élément mobile, comme expliqué aux figures 2A à 2C.

On rapproche alors mutuellement l'élément fixe et l'élément mobile, et on fait basculer la cupule 20 au sein de son logement 16. Ce basculement peut être réalisé par l'intermédiaire d'une tige non représentée, formant palpateur, qui vient en contact avec le fond 24 de la cupule 20, depuis l'ouverture du logement 16 opposée à la vis pédiculaire 2.

Enfin, on rapproche l'élément intermédiaire 20 ainsi basculé, par rapport à chaque vis 2, de manière que chaque méplat 6 puisse permettre l'introduction d'une vis correspondante dans le volume intérieur 30.

Une fois le dispositif de l'invention placé dans la configuration de la figure 3B, on peut rapporter sur le méplat 6 un moyen de butée, avantageusement amovible, telle qu'une vis 36. Cette dernière, en limitant le pivotement de la tête 4 par rapport à la cupule 20, empêche cette tête de recouvrer sa position de la figure 3, ce qui évite toute désolidarisation intempestive entre le cupule 20 et la vis 2.

L'invention n'est pas limitée à l'exemple décrit et représenté.

Ainsi, la cupule intermédiaire 20 peut être reçue dans un logement dont est équipée la vis pédiculaire, et non pas l'élément mobile. Dans ces conditions, l'élément mobile possède alors une tête sphérique, analogue à celle 4, apte à être introduite dans le volume intérieur de la cupule intermédiaire.

Par ailleurs, la vis 2, l'élément mobile 10 et la cupule intermédiaire 20 sont susceptibles de présenter d'autres agencements, tels que ceux décrits dans la demande de brevet français FR-A-2 810 873, déposée le 30 juin 2000 par le présent Demandeur, ainsi que ceux décrits dans la demande de brevet internationale WO-A-0 200 124, déposée le 29 juin 2001 par le présent Demandeur.

L'invention permet de réaliser les objectifs précédemment mentionnés.

En effet, les différents éléments constitutifs du dispositif de liaison intervertébral de l'invention possèdent une structure relativement simple.

L'assemblage de ces éléments est particulièrement aisé pour le chirurgien. En effet, l'élément intermédiaire peut être introduit par impaction dans le volume intérieur de l'élément mobile, ou de l'élément fixe.

Puis, la présence de l'élément intermédiaire autorise le montage mutuel des éléments fixe et mobile, même s'il n'existe pratiquement aucun débattement en rotation entre ces deux éléments.

En outre, il est à noter que, quand bien même l'élément intermédiaire est déformable, ce qui en facilite le montage, il devient sensiblement rigide une fois introduit dans son logement. Cette rigidité, qui lui est conférée par les parois rigides de ce logement, assure une stabilité satisfaisante au dispositif, une fois implanté.

Ainsi, ce dernier possède une résistance élevée à l'égard des contraintes mécaniques, s'exerçant notamment en traction. Par ailleurs, la présence de l'élément intermédiaire permet de transmettre, seulement dans une très faible mesure, les éventuels efforts auxquels est soumis le dispositif de liaison de l'invention.

## Revendications

1. Dispositif de liaison intervertébral, destiné à relier au moins deux vertèbres entre elles, comprenant :
- au moins un élément fixe (2), destiné à être rendu solidaire d'une vertèbre ou du sacrum,
- au moins un élément mobile de liaison (10), apte à se déplacer par rapport au ou à chaque élément fixe (2),
- ainsi qu'au moins un élément intermédiaire (20), permettant l'articulation du ou de chaque élément mobile par rapport au ou à chaque élément fixe,
- le ou chaque élément intermédiaire étant reçu, en service, dans un volume intérieur (16) de l'élément mobile (10), ou bien de l'élément fixe, cet élément intermédiaire étant déformable, de manière à pouvoir être introduit par impaction dans ce volume intérieur,
- l'élément fixe (2), ou bien l'élément mobile, étant reçu au moins partiellement, en service, dans un volume intérieur (30) de l'élément intermédiaire (20), **caractérisé en ce que** l'élément fixe (2) ou bien l'élément mobile présente, avec l'élément intermédiaire, une position mutuelle d'utilisation (figure 3B), dans laquelle cet élément fixe ou bien cet élément mobile possède trois degrés de liberté en rotation, mais se trouve lié en translation, par rapport à l'élément intermédiaire, et une position mutuelle d'introduction (figure 3A), dans laquelle cet élément fixe, ou bien cet élément mobile, possède trois degrés de liberté en rotation et en translation par rapport à cet élément intermédiaire, ladite introduction étant permise par un méplat équatorial (6) ménagé sur une tête sphérique (4) de l'élément fixe (2) ou de l'élément mobile (10), ledit méplat équatorial (6) n'étant pas perpendiculaire à l'axe principal (A) de l'élément fixe (2) ou de l'élément mobile (10) respectivement, et **en ce que** ledit dispositif est dépourvu de bague rigide qui serait rapportée au voisinage du débouché du volume intérieur (30) de l'élément intermédiaire (20).

2. Dispositif de liaison selon la revendication 1, **caractérisé en ce que** l'élément intermédiaire affecte la forme d'une cupule (20).

3. Dispositif de liaison selon la revendication 2, **caractérisé en ce que** le volume intérieur (30) de la cupule intermédiaire (20) est bordé par une surface sphérique tronquée (28).

4. Dispositif de liaison selon la revendication 3, **caractérisé en ce que** la cupule intermédiaire possède une surface extérieure sphérique tronquée (26), concentrique à la surface intérieure (28).

5. Dispositif de liaison selon la revendication 4, **caractérisé en ce que** les surfaces intérieure (28) et extérieure (26) définissent une paroi (22) de la cupule intermédiaire (20).

6. Dispositif de liaison selon la revendication 5, **caractérisé en ce que** l'épaisseur (e) de la paroi (22) est comprise entre 0,5 et 3 mm, de préférence entre 1 et 1,5 mm.

7. Dispositif de liaison selon l'une quelconque des revendications précédentes; **caractérisé en ce que** l'élément intermédiaire (20) est réalisé en polyéthylène.

## Claims

1. Inter-vertebral connection device designed to connect at least two vertebrae together, which involves:
- at least one fixed element (2), designed to be solidly fixed to a vertebra or to the sacrum,
- at least one mobile linking element (10) able to move in relation to the or each fixed element (2),
- as well as at least one intermediate element (20) which allows the or each mobile element to articulate in relation to the or each fixed element,
- the or each intermediate element being inserted, in service, into an internal space (16) in the mobile element 10, or in the fixed element, with this intermediate element being capable of undergoing deformation, so that it may be inserted by impaction into this internal space.
- the fixed element (2), or the moving element being at least partially inserted in service into an internal space (30) in the intermediate element (20), **characterised by** the fact that the fixed element (2) or the mobile element takes up a mutual position in use (figure 3B) with the intermediate element, in which this fixed element or this mobile element possesses three degrees of freedom in rotation, but is fixed in translation relative to the intermediate element, and a mutual position during insertion (figure 3A), in which this fixed element or this mobile element possesses three degrees of freedom of rotation and translation relative to this intermediate element, with the aforementioned insertion being permitted through an equatorial flat section (6) formed on a spherical head (4) of the fixed element (2), or of the mobile element (10), with the aforementioned equatorial flat section (6) not being perpendicular to the main axis (A) through the fixed element (2) or through the mobile element (10) respectively, and by the fact that the aforementioned device has no rigid ring which could be added in the vicinity of the opening of the internal space (30) of the intermediate element.

2. Connection device as described in claim 1, **characterised by** the fact that the intermediate element is in the form of a cup (20).

3. Connection device as described in claim 2, **characterised by** the fact that the edge of the internal space of the intermediate cup (20) is a truncated spherical surface (28).

4. Connection device as described in claim 3, **characterised by** the fact that the intermediate cup has an external truncated spherical surface (26) which is concentric with the internal surface (28).

5. Connection device as described in claim 4, **characterised by** the fact that the internal (28) and external (25) surfaces define a side wall (22) of the intermediate cup (20).

6. Connection device as described in claim 5, **characterised by** the fact that the thickness (e) of the side wall (22) is between 0.5 and 3 mm, preferably between 1 and 1.5 mm.

7. Connection device as described in any of the preceding claims whatsoever, **characterised by** the fact that the intermediate element (20) is made of polyethylene.

## Patentansprüche

1. Wirbelverbindungsvorrichtung, die dazu bestimmt ist, mindestens zwei Wirbel miteinander zu verbinden, mit:
- wenigstens einem festen Element (2), das dazu bestimmt ist, mit einem Wirbel oder dem Kreuzbein verbunden zu werden,
- wenigstens einem beweglichen Verbindungselement (10), das dazu in der Lage ist, sich in bezug auf das oder jedes feste Element (2) zu verlagern,
- und wenigstens einem Zwischenelement (20), das eine Gelenkbewegung des oder jedes beweglichen Elements in bezug auf das oder jedes feste Element ermöglicht,
- wobei das oder jedes Zwischenelement im Gebrauch in einem inneren Volumen (16) des beweglichen Elements (10) oder auch des festen Elements aufgenommen ist, und dieses Zwischenelement so verformbar ist, daß es durch Einschlagen in das innere Volumen eingeführt werden kann,
- wobei das feste Element (2) oder auch das bewegliche Element im Gebrauch zumindest teilweise in einem inneren Volumen (30) des Zwischenelements aufgenommen ist, **dadurch gekennzeichnet, daß** das feste Element (2) oder auch das bewegliche Element mit dem Zwischenelement eine relative Gebrauchsstellung (Figur 3B) bietet, in der dieses feste Element oder auch das bewegliche Element drei Freiheitsgrade der Rotation besitzt, jedoch translationsfest mit dem Zwischenelement verbunden ist, sowie eine gegenseitige Einführungsposition (Figur 3A), in der dieses feste Element oder auch das bewegliche Element drei Freiheitsgrade der Rotation und der Translation in bezug auf dieses Zwischenelement besitzt, wobei das genannte Einführen ermöglicht wird durch eine äquatoriale Abflachung (6), die an einem sphärischen Kopf des festen Elements (2) oder auch des beweglichen Elements (10) ausgebildet ist, wobei diese äquatoriale Abflachung (6) nicht senkrecht auf der Hauptachse (A) des festen Elements (2) bzw. des beweglichen Elements (10) steht, und daß die genannte Vorrichtung einen starren Ring aufweist, der in der Nähe der Mündung des inneren Volumens (30) des Zwischenelements (20) angeordnet wird.

2. Verbindungsvorrichtung nach Anspruch 1, **dadurch gekennzeichnet, daß** das Zwischenelement die Form einer Kuppel (20) annimmt.

3. Verbindungsvorrichtung nach Anspruch 2, **dadurch gekennzeichnet, daß** das innere Volumen (30) der das Zwischenelement bildenden Kuppel (20) durch eine Kugelsegmentfläche (28) begrenzt wird.

4. Verbindungsvorrichtung nach Anspruch 3, **dadurch gekennzeichnet, daß** die das Zwischenelement bildende Kuppel eine kugelsegmentförmige äußere Oberfläche (26) konzentrisch zu der inneren Oberfläche (28) hat.

5. Verbindungsvorrichtung nach Anspruch 4, **dadurch gekennzeichnet**, daβ die inneren und äußeren Oberflächen (28, 26) eine Wand (22) der das Zwischenelement bildenden Kuppel (20) definieren.

6. Verbindungsvorrichtung nach Anspruch 5, **dadurch gekennzeichnet, daß** die Dicke (e) der Wand (22) zwischen 0,5 und 3 mm vorzugsweise zwischen 1 und 1,5 mm beträgt.

7. Verbindungsvorrichtung nach einem der vorstehenden Ansprüche, **dadurch gekennzeichnet, daß** das Zwischenelement (20) aus Polyethylen hergestellt ist.
